# EUROPEAN PATENT APPLICATION

(11) **EP 2 009 092 A1**
(43) Date of publication of application: **31.12.2008**
(21) Application number: 07110949.0
(22) Date of filing: 25.06.2007
(51) Int. Cl.: C12N 1/12, B01D 53/04, B01J 20/02

(54) **Method for producing aquatic biomass**

(71) Applicant: BIOeCON International Holding N.V., Curaçao (AN)
(72) Inventor: O'Connor, Paul, 3871 KM Hoevelaken (NL)
(74) Representative: Rasser, Jacobus Cornelis

(57) **Abstract**

Disclosed is a plant for producing aquatic biomass. The plant comprises a pond adapted for growing aquatic biomass, a system for providing CO₂ to said pond, and a CO₂ capturing material capable of reversibly capturing CO₂. The CO₂ capturing makes it possible to uncouple the production of the aquatic biomass from the availability of CO₂, both in terms of time and location. For example, CO₂ produced during the night may be stored, and supplied to the pond during sunlight hours. Or CO₂ produced at a remote location may be stored and transported to the pond.

## Description

### BACKGROUND OF THE INVENTION

### 1. Field of the Invention

The invention relates generally to the production of liquid fuels from aquatic biomass, and more particularly to improved processes for producing aquatic biomass and for converting aquatic biomass to bio-oil, which can be used as a burning fuel, or in turn can be refined to fuels for internal combustion engines.

### 2. Description of the Related Art

Aquatic plants, such as algae, are a source of lipids, such as triglycerides and aliphatic hydrocarbons; carbohydrates, such as lignin; and proteins.

The advantage of using micro algae is that they have very high growth rates, utilize a large fraction of the solar energy (up to 10% of the solar energy), and can grow in conditions that are not favorable for terrestrial biomass growth.

The U.S. Department of Energy funded a program to develop renewable transportation fuels from algae, and the results of this program are reported by Sheehan et al. [1] Over 3000 strains of micro algae were collected as part of this program. Micro algae are one of the most primitive forms of plants and are microscopic photosynthetic organisms. While the photosynthesis mechanism in algae is similar to other plant material, they can convert much more of their solar energy into cellular structure (up to 10% instead of maximum 1% by terrestrial sources).

Macro algae are commonly known as seaweed. Both micro algae and macro algae are fast-growing marine and freshwater plants. Commercial production of triglycerides from micro algae has been estimated to be 72 000 L/ha-year (390 boe/ha-year), and it has been estimated that rates as high as 130 000 L/ha-year (700 boe/ha-year) could be accomplished.

This means that algae have triglyceride production rates 45-220 times higher than terrestrial biomass. Other estimates indicate that 2000 ha of land would be required to produce 1 EJ/year of fuel with micro algae. For comparison, the U.S. consumed 42 EJ of petroleum products in 2003.

Micro algae are categorized into four major classes in terms of their abundance: diatoms, green algae, blue-green algae, and golden algae. Micro algae can contain from 7 to 60 dry wt % triglycerides.

Pilot plant tests [2], conducted over a six-year period, demonstrate that micro algae could be produced at productivity rates as high as 500 kg algae/ha in a 1000 m2 pond for a single day. The ponds were an open face shallow water design where the water and algae are circulated around the pond. Nutrients and CO2 were continually added to the algae pond. The productivity was dependent on temperature and sunlight, which varied over the course of the experiments. Ideally, algae could be produced on algae farms in open, shallow ponds where a waste source of CO₂, for example, from a fossil fuel power plant, could be efficiently bubbled into the ponds and captured by the algae.

The current limitation of micro algae is the high production cost. The total biomass algae cost is in the order of 200 to 300 $/metric ton, which is considerably higher than the cost of lignocellulosic biomass (less than $40/metric ton). The cost for CO₂ is 20-30% of the total cost, and using waste CO₂ from fossil fuel power plants would decrease the cost of algae production.

The conclusions from the cost analysis [1,2] is that alternative engineering designs for micro algae production would not significantly reduce the cost of micro algae production. The limiting factor in cost analysis is micro algae cultivation issues, and according to Sheehah [1] future research work should focus on the biological issues regarding micro algae production. Micro algae cultivation issues are limited by the availability of water, CO₂, sun light, and flat land. The development of low-cost harvesting processes can also significantly reduce the cost of algae.

Thus, there is a particular need for improving the efficiency of growing aquatic biomass, and of the conversion of aquatic biomass to bio-oil.

### BRIEF SUMMARY OF THE INVENTION

The present invention addresses these problems by providing a plant for producing aquatic biomass comprising:
a) a pond adapted for growing aquatic biomass;
b) a system for providing CO₂ to said pond;
c) a CO₂ capturing material capable of reversibly capturing CO₂.

### DESCRIPTION OF ILLUSTRATIVE EMBODIMENTS

The following is a description of certain embodiments of the invention, given by way of example only.

The present invention focuses on two main factors responsible for the high cost of liquid fuel from aquatic biomass, the high cost of CO₂, and the high cost of converting aquatic biomass to bio-oil.

As used herein the term "aquatic biomass" refers to biomass produced in an aquatic environment by a photosynthesis process. The most common form of aquatic biomass is obtained from growing algae.

As compared to land-based plants, algae are highly efficient in the photosynthesis process. In photosynthesis, CO₂ is converted to hydrocarbons under the influence of sun light. The common catalyst used by plants in the photosynthesis process is chlorophyll. Plants do not need direct sun light for the photosynthesis process; the process takes place in indirect sun light, such as when the sky is overcast, be it more slowly than in direct sun light.

Algae may be grown in shallow ponds (e.g., between 10 cm and 1 m deep), preferably in sunny climates. Because many species of algae may be grown in salt water, it is often advantageous to use sea water, so that the crop does not consume precious fresh water supplies.

When there is enough available sun light, the availability of CO₂ generally is the rate determining step on the production of aquatic biomass. Although there is an unlimited supply of CO₂ in the earth's atmosphere, the concentration of CO₂ in ambient air is low. The gas is also poorly soluble in water.

It has been proposed to increase the growth rate of aquatic biomass by bubbling CO₂ through an algae growing pond. Although this technique significantly increases the production of aquatic biomass, the cost of CO₂ gas considerably adds to the cost of aquatic biomass so produced.

It is possible to use for the production of aquatic biomass CO₂ produced in the combustion of fossil fuels, such as in coal, oil or gas-fired power plants. However, it is rarely possible to construct algae growing ponds of a meaningful size in the immediate vicinity of a power plant. Also, many power plants are not located in areas that have the required amount of sunshine required for the economic production of aquatic biomass. Even if all these requirements are met, power plants tend to produce most of their CO₂ by-product at night and during the winter time, that is, when the demand for CO₂ from the algae-growing ponds is low.

There is therefore a need for a process for producing aquatic biomass that has a reduced dependency on the time and location of the production of CO₂ in the combustion of fossil fuel.

This is addressed by the present invention, which provides a plant for producing aquatic biomass comprising:
a) a pond adapted for growing aquatic biomass;
b) a system for providing CO₂ to said pond;
c) a CO₂ capturing material capable of reversibly capturing CO₂

The reversible capture of CO₂ may be based on temperature. Materials particularly suitable for use in element c) are those that capture CO₂ when contacted with CO₂ at a relatively low temperature, for example a temperature below 200 °C, and release CO₂ when heated to a more elevated temperature, for example a temperature above 250 °C.

Preferred CO₂ capturing materials are those comprising an inorganic oxide. Suitable examples include natural and synthetic clays; oxides and hydroxides of aluminum, magnesium, calcium; alumina/magnesia mixtures; meixnerites; hydrotalcite and hydrotalcite-like materials; and mixtures thereof.

The CO₂ capturing material is loaded with CO₂ at or near a location where CO₂ is produced, for example as a by-product of some other process, such as the generation of electricity. After loading with CO₂ the material is shipped to the plant for producing aquatic biomass, where it is charged to a suitable reactor for release of the CO₂. Importantly, the material may temporarily be stored until the CO₂ demand of the plant justifies its use.

Although the invention permits the transportation of CO₂ over any distance, in the form of the CO₂ capturing material loaded with CO₂, it will be understood that transportation distances are preferably kept short.

The invention may also use CO₂ produced in the combustion of a renewable resource, such a bio-fuel. In this case the process results in a net reduction of the output of CO₂, thereby off-setting CO₂ production from fossil fuels elsewhere on the planet. The off-set results in valuable carbon credits, which may be traded in the market for such credits.

The pond adapted for growing aquatic biomass preferably has a depth of from 10 to 100 cm. Depending on the algae species being grown, the pond may be filled with sea water or fresh water. The use of sea water is preferred, as its use does not divert precious fresh water supplies.

CO₂ is released from the CO₂ capturing material by, for example, heating the capturing material in a suitable reactor to a temperature at which the captured CO₂ is released. The capturing material is hereby regenerated. The regenerated capturing material may be shipped back to the CO₂-producing location, for re-use.

CO₂ produced in the reactor is pumped to the pond for growing aquatic biomass, and bubbled through the water contained in the pond through suitable nozzles. Preferably the nozzles are located near the bottom of the pond.

It is desirable to tune the amount of CO₂ provided to the pond to the amount the algae are capable of converting. On an annual basis it is estimated that a pond may produce from 100 to 400 metric tons of aquatic biomass per hectare (10⁴ m²) per year. This corresponds to 10 to 40 kg per m² per year. About two thirds of this mass comes from CO₂ (the other one third comes from water consumed in the photosynthesis process). Therefore a pond consumes from about 6.7 to about 27 kg CO₂ per m² per year.

The consumption of CO₂ per hour fluctuates with the amount of sunshine available at any given point in time, and with the amount of algae present in the pond. The skilled person will be able to estimate the CO₂ consumption. It is possible to provide a computer-control based process, which uses the brightness of the sunlight and the amount of biomass in the water (based on, for example, turbidity) as input parameters, and which provides the CO₂ demand as an output parameter.

The invention further comprises a method for producing aquatic biomass comprising the steps of:
a) providing a pond containing water and suitable nutrients for growing aquatic biomass;
b) providing algae for growing in the pond;
c) providing light shining on the pond;
d) providing CO₂ to the pond from a CO₂ capturing material capable of reversibly capturing CO₂.

Preferably the algae used in step b) comprise micro-algae.

The light used in step c) preferably is natural sunlight.

Preferred CO₂ capturing materials are those comprising an inorganic oxide. Suitable examples include natural and synthetic clays; oxides and hydroxides of aluminum, magnesium, calcium; alumina/magnesia mixtures; meixnerites; hydrotalcite and hydrotalcite-like materials; and mixtures thereof.

Step d) preferably comprises the steps of:
(i) contacting the CO₂ capturing material with CO₂ at a temperature conducive to CO₂ capture by the CO₂ capturing material;
(ii) heating the CO₂ capturing material to cause release of the CO₂ captured by the CO₂ capturing material.

In a preferred embodiment the CO₂ capturing material is contacted with CO₂ produced in the combustion of a fossil fuel. Such combustion may take place in, for example, a power plant, a plant for liquefying or gasifying coal, a refinery, and the like.

References:

[1] Sheehan, J; Dunahay, T; Benneman, J. Roessler, P. A Look Back at the US Department of Energy's Aquatic Species Program - Biodiesel from Algae; Report No. NREL/TP-580-24190; National Renewable Energy Laboratory: Golden, CO 1998; http://www.osti.gov/bridge

[2] Hill, A.M.; Feinberg, D.A., Fuel Products from Microalgae; Report No. SERI/TP-231-2348; National Renewable Energy Laboratory: Golden, CO, 1984

## Claims

1. A plant for producing aquatic biomass comprising:
a) a pond adapted for growing aquatic biomass;
b) a system for providing CO₂ to said pond;
c) a CO₂ capturing material capable of reversibly capturing CO₂.

2. The plant of claim 1 wherein the CO₂ capturing material captures CO₂ when contacted with CO₂ at temperatures below about 200 °C.

3. The plant of claim 1 or 2 wherein the CO₂ capturing material releases CO₂ when heated at a temperature above about 250 °C.

4. The plant of any one of the preceding claims wherein the CO₂ capturing material comprises an inorganic oxide.

5. The plant of claim 4 wherein the CO₂ capturing material is selected from the group consisting of natural clays; synthetic clays; aluminum oxides; aluminum hydroxides; magnesium oxides; magnesium hydroxides; calcium oxides; calcium hydroxides; alumina-magnesia; Meixnerite; hydrotalcite; hydrotalcite-like materials; and mixtures thereof.

6. The plant of any one of the preceding claims wherein the pond is filled with seawater at a depth of from 10 to 100 cm.

7. The plant of any one of the preceding claims wherein the system for providing CO₂ to the pond comprises nozzles submerged in water.

8. The plant of any one of the preceding claims wherein the system for providing CO₂ to the pond is capable of providing between 6.7 and 27 kg of CO₂ per hour and per m² of pond surface area.

9. A method for producing aquatic biomass comprising the steps of:
a) providing a pond containing water and suitable nutrients for growing aquatic biomass;
b) providing algae for growing in the pond;
c) providing light shining on the pond;
d) providing CO₂ to the pond from a CO₂ capturing material capable of reversibly capturing CO₂.

10. The method of claim 9 wherein the algae comprise micro algae.

11. The method of claim 9 or 10 wherein the light is natural sunlight.

12. The method of any one of claims 9-11 wherein the CO₂ capturing material comprises an inorganic oxide.

13. The method of any one of claims 9 - 12 wherein the CO₂ capturing material is selected from the group consisting of natural clays; synthetic clays; aluminum oxides; aluminum hydroxides; magnesium oxides; magnesium hydroxides; calcium oxides; calcium hydroxides; alumina-magnesia; Meixnerite; hydrotalcite; hydrotalcite-like materials; and mixtures thereof.

14. The method of any one of claims 9 - 13 wherein step d) comprises the steps of:
(i) contacting the CO₂ capturing material with CO₂ at a temperature conducive to CO₂ capture by the CO₂ capturing material;
(ii) heating the CO₂ capturing material to cause release of the CO₂ captured by the CO₂ capturing material.

15. The method of claim 14 wherein, in step (i), the CO₂ capturing material is contacted with CO₂ produced in the combustion of a fossil fuel.

16. The method of claim 15 wherein the CO₂ is produced in a refinery, a plant for liquefying or gasifying coal, or a power plant.
